# EUROPEAN PATENT APPLICATION

(11) **EP 4 834 426 A1**
(43) Date of publication of application: **04.11.2026**
(21) Application number: 24913797.7
(22) Date of filing: 12.12.2024
(51) Int. Cl.: A61N 7/02, A61N 5/06, A61N 5/067, A61N 1/40, A61N 1/32, A61H 9/00, A61H 23/02, A61N 7/00

(54) **SKIN CARE DEVICE INCLUDING LIGHTWEIGHT HANDPIECE**

(30) Priority: 26.12.2023 KR 20230191278
(71) Applicant: CLASSYS INC., Seoul 06220 (KR)
(72) Inventor: LEE, Chung Keun, Siheung-si Gyeonggi-do 14995 (KR); SHIN, Yeon Seok, Hwaseong-si Gyeonggi-do 18342 (KR)
(74) Representative: Witte, Weller & Partner Patentanwälte mbB
(86) International application number: PCT/KR2024/096777
(87) International publication number: WO 2025/143993

(57) **Abstract**

The present invention relates to a skin care device including a lightweight handpiece. The skin care device comprises: a handpiece for skin treatment which comes into contact with the skin and stimulates the skin; a control body which is connected to the handpiece for skin treatment through a connection cable body and controls the operation of the handpiece for skin treatment; a driving force generating unit which is mounted inside the control body and generates a driving force; a handpiece operating body which is provided in the handpiece for skin treatment and operates by receiving the driving force from the driving force generating unit; and a driving force transmission unit for transmitting the driving force of the driving force generating unit to the handpiece operating body through the inside of the connection cable body. Since the driving force generating unit for operating the handpiece operating body is mounted on the control body, the weight of the handpiece for skin treatment is reduced, thereby greatly improving the convenience of a practitioner and the satisfaction of treatment during treatment, and simplifying the structure of the handpiece for skin treatment.

## Description

### Technical Field

The present disclosure relates to a skin care device including a lightweight handpiece. More particularly, the present disclosure relates to a skin care device including a lightweight handpiece in which a driving force generating unit for the handpiece is mounted in a control body.

### Background Art

Recently, interest in skin beauty has been increasing, and various skin care devices have been developed.

Skin care devices have been proposed in various structures, such as a skin treatment device using ultrasound, a skin treatment device using a laser, a skin treatment device using RF energy, a skin cleaning device for face lifting treatment or skin tightening treatment, a cooling lipolysis treatment device, and so on.

A skin care device using ultrasound applies high intensity focused ultrasound by using a handpiece for generating ultrasound, thereby generating heat at a focal region of skin tissue positioned at a predetermined depth from a skin surface. The generated heat induces degeneration and regeneration of skin tissue, thereby producing a cosmetic skin-care effect, such as wrinkle reduction.

In addition, a skin treatment device using a laser irradiates the skin with a laser in dots having a predetermined size by using a handpiece for generating a laser, and treats the skin of a patient in a manner that damages skin tissue by applying heat with the laser only to a treatment target region. The skin treatment device using the laser is used for various purposes, such as preventing hair loss or promoting hair growth, skin peeling, skin regeneration, whitening, removing wrinkles or spots, removing melasma, and so on.

In addition, a skin treatment device using RF energy performs treatment by bringing an RF electrode of a handpiece into close contact with the skin of a patient and applying electrical power to the RF electrode, thereby delivering RF energy to the skin. The skin treatment device using RF energy is variously used for skin remodeling/resurfacing, wrinkle removal, and treatment of sebaceous glands, hair follicles, adipose tissue, and spider veins through contraction or a wound healing reaction of collagen in a lower dermis.

In addition, a skin cleaning device treats skin of a patient in a manner that discharges a cleaning solution for allowing functional cosmetic ingredients to penetrate into skin to a skin surface by using a handpiece for skin cleaning and polishes the skin surface.

In addition, a cryolipolysis device for cooling lipolysis treatment is used in a state in which a cooling cup for cooling a treatment region of a treatment subject is mounted on one handpiece.

The cooling cup is divided into a suction type that suctions and cools skin of the treatment subject and a non-suction type that does not suction skin of the treatment subject, and performance of the cooling cup varies according to the type and number of thermoelectric elements attached to the cooling cup.

A practitioner may increase the effect of cooling lipolysis treatment by selecting a cooling cup most suitable for the treatment subject from among various types of cooling cups according to an obesity state or a treatment region of the treatment subject and performing treatment.

Meanwhile, a skin care device includes a handpiece for skin treatment which comes into contact with the skin of a subject to be treated and which performs treatment for stimulating the skin, and includes a control body which is connected to the handpiece for skin treatment by a cable body and which is configured to control operation of the handpiece for generating ultrasound.

The handpiece for skin treatment is provided with a skin stimulation generating unit configured to stimulate skin, and is provided with a driving force generating unit for moving or rotating the skin stimulation generating unit.

In addition, the handpiece for skin treatment may be provided with a driving force generating unit for generating vibration for skin massage or the like.

For example, a handpiece for generating ultrasound or a handpiece for generating a laser is provided with a driving motor that linearly or rotationally moves an ultrasound generating unit or a laser generating unit with a preset radius.

In addition, a handpiece for skin cleaning is provided with a driving motor for rotating a polishing pad.

In addition, a handpiece for skin cleaning, a handpiece provided with an RF electrode, a cooling cup, and a handpiece for skin cleaning are provided with a vibration motor or the like for generating vibration for skin massage.

That is, in a conventional skin care device, a driving force generating unit, such as a driving motor or a vibration motor, is positioned in a handpiece for skin treatment, and a board provided with a driving circuit and a sensing circuit for controlling operation of the driving force generating unit, a gear structure for transmitting rotational force of the motor, and so on are included. Therefore, there is a problem in that the size and shape of the handpiece for skin treatment are limited.

In addition, since the handpiece for skin treatment includes the motor, the board, the gear structure for transmitting rotational force of the motor, and so on, the handpiece for skin treatment is heavy. Furthermore, there is a problem in that vibration generated when the motor operates during treatment places strain on a wrist of a practitioner during treatment and causes inconvenience in a treatment operation.

A patent document related to the present disclosure includes Korean Patent No. 10-1307551, entitled "HANDPIECE OF ULTRASONIC DEVICE" (registered on September 5, 2013).

In addition, a patent document related to the present disclosure includes Korean Patent No. 10-2038941, entitled "HANDPIECE FOR LASER OPERATION AND FAT REDUCTION APPARATUS WITH THE SAME" (registered on October 25, 2019).

### Disclosure

### Technical Problem

An objective of the present disclosure is to provide a skin care device including a lightweight handpiece, the skin care device being configured such that a driving force generating unit for operating an operating body in a handpiece is mounted in a control body, thereby simplifying the structure and reducing weight of the handpiece for skin treatment.

### Technical Solution

In order to achieve the objective described above, according to an aspect of the present disclosure, there is provided a skin care device including a lightweight handpiece, the skin care device including: a handpiece for skin treatment, the handpiece being configured to come into contact with skin and to stimulate the skin; a control body connected to the handpiece for skin treatment by a connection cable body and configured to control operation of the handpiece for skin treatment; a driving force generating unit mounted inside the control body and configured to generate a driving force; a handpiece operating body provided in the handpiece for skin treatment and configured to be operated by receiving the driving force of the driving force generating unit; and a driving force transmission unit which passes through an inside of the connection cable body and which is configured to transmit the driving force of the driving force generating unit to the handpiece operating body.

In the present disclosure, the driving force generating unit may include a rotation motor unit configured to generate rotational force, and the driving force transmission unit may include a rotational force transmission unit configured to transmit the rotational force of the rotation motor unit to the handpiece operating body.

In the present disclosure, the rotational force transmission unit may include: a first pulley member rotatably positioned inside the control body and configured to be rotated by receiving the rotational force of the rotation motor unit; a second pulley member rotatably positioned inside the handpiece for skin treatment; and a belt structure having a first end portion wound around the first pulley member and having a second end portion wound around the second pulley member.

In the present disclosure, the belt structure may be any one of a wire-type belt, a strip-type belt, a V-belt, and a chain.

In the present disclosure, the rotational force transmission unit may further include a belt movement guide tube part which is fixed inside the connection cable body and through which the belt structure passes and moves, the belt movement guide tube part being configured to support a position of the belt structure.

In the present disclosure, the connection cable body may include: an outer skin part surrounding an operation control cable and the belt structure that pass through the inside of the connection cable body; and a filler formed of an elastic material, the filler being filled in the outer skin part and supporting positions of the operation control cable and the belt movement guide tube part.

In the present disclosure, the handpiece for skin treatment may include: a cartridge housing part provided with a skin stimulation generating unit configured to stimulate the skin; and a handpiece body casing part to which the cartridge housing part is detachably coupled. Furthermore, the driving force transmission unit may further include a cartridge driving unit positioned in the cartridge housing part, the cartridge driving unit being configured to receive the rotational force from the rotational force transmission unit and to linearly move or rotate the handpiece operating body.

In the present disclosure, an operating shaft of the cartridge driving unit may be detachably coupled to an operating shaft of the rotational force transmission unit.

In the present disclosure, the handpiece operating body may include the skin stimulation generating unit positioned in the cartridge housing part, and the cartridge driving unit may include a linear movement conversion unit configured to linearly move the skin stimulation generating unit.

In the present disclosure, the linear movement conversion unit may include: a screw member rotatably positioned in the handpiece body casing part and configured to be rotated by receiving rotational force of a second pulley member; and a slider through which the screw member passes and to which the screw member is screw-coupled, the slider being configured to be linearly moved by rotation of the screw member and being connected to the skin stimulation generating unit.

In the present disclosure, the linear movement conversion unit may further include a movement guide pin member which is positioned to pass through the slider and which is configured to guide linear movement of the slider.

In the present disclosure, the rotational force transmission unit may further include a first rotation direction changing gear member and a second rotation direction changing gear member that are configured to change a rotation direction and to transmit the rotational force to the screw member.

In the present disclosure, the linear movement conversion unit may further include: a first bellows member having a first end portion fixed to an inner side surface of the cartridge housing part and having a second end portion fixed to a first end portion of the slider, the first bellows member being configured to be folded and unfolded by movement of the slider and having the screw member positioned therein; and a second bellows member having a second end portion fixed to the inner side surface of the cartridge housing part and having a first end portion fixed to a second end portion of the slider, the second bellows member being configured to be folded and unfolded by movement of the slider and having the screw member positioned therein.

### Advantageous Effects

The present disclosure has an effect of significantly increasing convenience for a practitioner and satisfaction with the treatment by mounting the driving force generating unit for operating the operating body in the handpiece in the control body and reducing weight of the handpiece for skin treatment.

In addition, the present disclosure has an effect of greatly increasing freedom in handpiece design since the handpiece for skin treatment may be designed without limitation in size and shape by simplifying a structure of the handpiece for skin treatment.

### Description of Drawings

FIG. 1 is a view illustrating an embodiment of a skin care device including a lightweight handpiece according to the present disclosure.
FIG. 2 is a cross-sectional view illustrating an embodiment of the skin care device including the lightweight handpiece according to the present disclosure.
FIG. 3 is an enlarged view of portion A in FIG. 2.
FIG. 4 is an enlarged view illustrating an embodiment of a connection cable body in an embodiment of the skin care device including the lightweight handpiece according to the present disclosure.

### *DESCRIPTION OF REFERENCE NUMERALS OF IMPORTANT PARTS

100: Handpiece for skin treatment 110: Cartridge housing part
110a: Window part 120: Handpiece body casing part
120a: Cartridge locking part 200: Control body
210: Connection cable body 211: Outer skin part
212: Operation control cable 213: Belt movement guide tube part
214: Filler 220: Main control unit
300: Driving force generating unit 310: Rotation motor unit
400: Handpiece operating body 410: Skin stimulation generating unit
411: Ultrasound generating unit 500: Driving force transmission unit
510: Rotational force transmission unit 511: First pulley member
512: Second pulley member 513: Belt structure
514: First rotation direction changing gear member
515: Second rotation direction changing gear member
520: Cartridge driving unit 521: Linear movement conversion unit
521a: Screw member 521b: Slider
521c: Movement guide pin member 521d: First bellows member
521e: Second bellows member 530: Shaft adapter part
531: Shaft coupling protrusion part 532: Shaft coupling insertion part

### Mode for Invention

Hereinafter, exemplary embodiments of the present disclosure will be described in detail with reference to the accompanying drawings. However, the present disclosure may be embodied in different forms and should not be construed as limited to the embodiments set forth herein. Rather, the embodiments introduced herein are provided so that disclosed contents will be thorough and complete, and will fully convey the scope of the present disclosure to those skilled in the art.

In the present specification, it will be understood that when an element is referred to as being "on" another element, it can be formed directly on the other element or intervening elements may be present. In addition, in the drawings, the shapes and the thicknesses of regions are exaggerated for clarity.

In addition, it will be also understood that although the terms first, second, third, and so on may be used herein to describe various elements, these elements should not be limited by these terms. These terms are only used to distinguish one element from another element. Thus, a first element in some embodiments may be termed a second element in other embodiments without departing from the teachings of the present disclosure. Embodiments explained and illustrated herein include their complementary counterparts. As used herein, the term "and/or" includes any and all combinations of one or more of the associated listed elements.

The singular expression also includes the plural meaning as long as it does not differently mean in the context. In addition, the terms "comprise", "have", and so on of the description are used to indicate that there are features, numbers, steps, elements, or combination thereof, and they should not exclude the possibilities of combination or addition of one or more features, numbers, operations, elements, or a combination thereof. Furthermore, it will be understood that when an element is referred to as being "connected" or "coupled" to another element, it may be directly connected or coupled to the other element or intervening elements may be present.

In addition, when detailed descriptions of related known functions or constitutions are considered to unnecessarily cloud the gist of the present disclosure in describing the present disclosure below, the detailed descriptions will not be included.

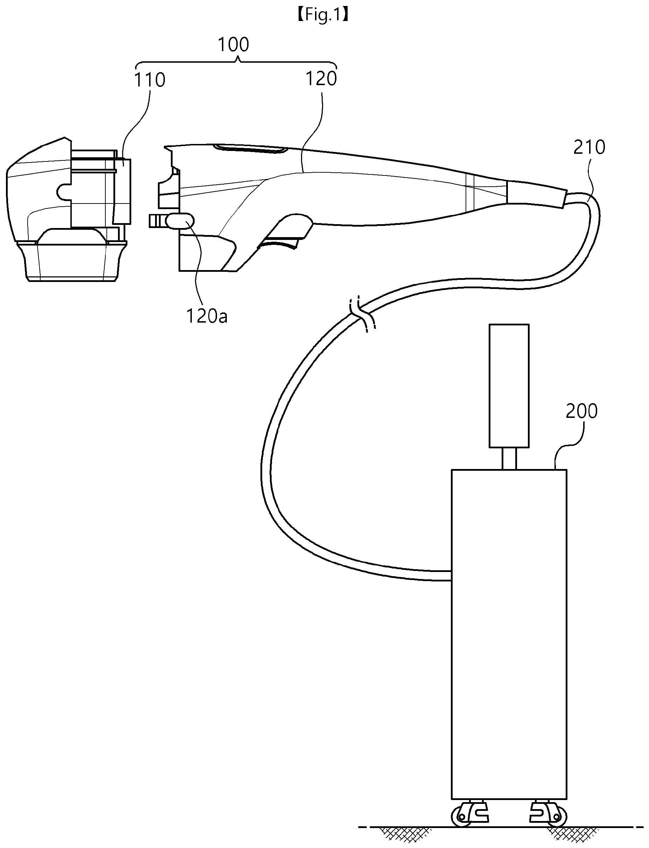

FIG. 1 is a view illustrating an embodiment of a skin care device including a lightweight handpiece according to the present disclosure.

An embodiment of the skin care device including the lightweight handpiece according to the present disclosure includes a handpiece 100 for skin treatment configured to come into contact with skin and to stimulate the skin, and includes a control body 200 connected to the handpiece 100 for skin treatment by a connection cable body 210 and configured to control operation of the handpiece 100 for skin treatment.

The handpiece 100 for skin treatment includes a skin stimulation generating unit 410 configured to stimulate skin.

For example, the skin stimulation generating unit 410 is any one of an ultrasound generating unit 411 configured to irradiate skin with ultrasound, a laser generating unit configured to irradiate skin with a laser, an RF electrode unit configured to come into contact with skin and to deliver RF energy to the skin, a cup part configured to come into contact with skin and to alternately generate negative pressure and positive pressure on the skin, and a skin massage unit configured to be rotated or vibrated in a state of contacting skin.

The skin stimulation generating unit 410 is known in a known skin beauty treatment device or a known skin care treatment device that stimulates skin to perform skin beauty treatment or skin care treatment, and a detailed description of the skin stimulation generating unit 410 is omitted.

That is, the skin care device including the lightweight handpiece according to the present disclosure is, for example, any one of a skin ultrasound generating device including a handpiece for ultrasound treatment, a skin laser generating device including a handpiece for laser treatment, a skin RF energy generating device including a handpiece for RF treatment, a negative pressure generating device including a handpiece for negative pressure treatment, and a known skin beauty device or a known skin care treatment device including a handpiece for skin massage.

The handpiece 100 for skin treatment includes a cartridge housing part 110 provided with the skin stimulation generating unit 410 configured to come into contact with skin and to stimulate the skin, and includes a handpiece body casing part 120 to which the cartridge housing part 110 is detachably coupled.

The cartridge housing part 110 is detachably coupled to the handpiece body casing part 120 as a consumable component. That is, when the skin stimulation generating unit 410 is used for a preset treatment time or a preset number of treatments, the cartridge housing part 110 reaches the end of its service life, becomes unusable for skin treatment, and is replaced.

For example, the cartridge housing part 110 is slidably moved on and coupled to a front end portion of the handpiece body casing part 120, and is locked by a cartridge locking part 120a at a final coupling position, thereby completing coupling.

The cartridge locking part 120a is a button-type locking part positioned on both sides of the handpiece body casing part 120, and uses a known configuration in which a wedge part for holding and supporting the cartridge housing part 110 is positioned on an elastically supported button part and locking is released when the button part is pressed, so that a detailed description of the cartridge locking part 120a will be omitted.

The control body 200 is connected to the handpiece 100 for skin treatment by the connection cable body 210 including a plurality of electric wires or circulation hoses, may control operation of the handpiece 100 for skin treatment, and may circulate a liquid, such as cooling water or a liquid medium for transmitting ultrasound, in the cartridge housing part 110.

This control structure is applied according to the type of the skin stimulation generating unit 410 in skin care devices, such as a skin laser generating device, a skin RF energy generating device, a negative pressure generating device, and a skin beauty device, and may be variously modified and implemented.

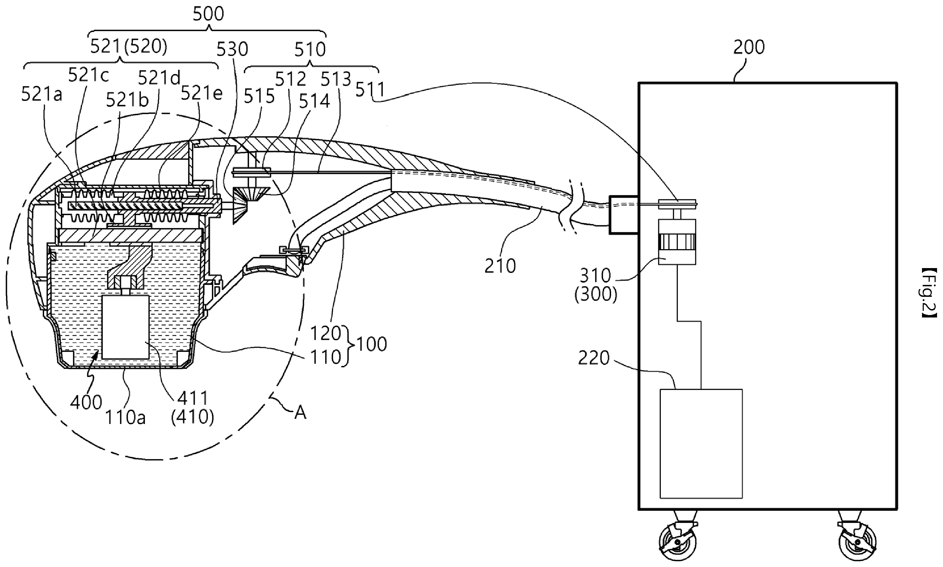

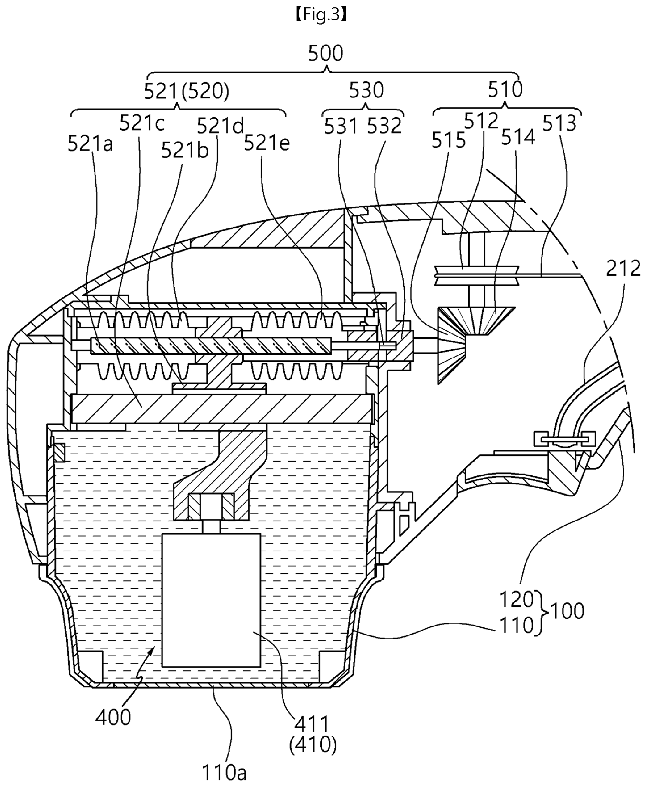

FIG. 2 is a cross-sectional view illustrating an embodiment of the skin care device including the lightweight handpiece according to the present disclosure, and FIG. 3 is an enlarged view of portion A in FIG. 2.

Referring to FIG. 2 and FIG. 3, an embodiment of the skin care device including the lightweight handpiece according to the present disclosure further includes a driving force generating unit 300 mounted inside the control body 200 and configured to generate a driving force, a handpiece operating body 400 provided in the handpiece 100 for skin treatment and configured to be operated by receiving the driving force of the driving force generating unit 300, and a driving force transmission unit 500 which passes through an inside of the connection cable body 210 and which is configured to transmit the driving force of the driving force generating unit 300 to the handpiece operating body 400.

The driving force generating unit 300 includes a rotation motor unit 310 configured to generate rotational force, and includes the driving force transmission unit 500 includes a rotational force transmission unit 510 configured to transmit the rotational force of the driving force generating unit 300 to the handpiece operating body 400.

The rotation motor unit 310 is connected to a main control unit 220 positioned in the control body 200 so that operation of the rotation motor unit 310 is controlled, and the main control unit 220 is electrically connected to the skin stimulation generating unit 410 of the handpiece 100 for skin treatment through the connection cable body 210 and is configured to control operation of the skin stimulation generating unit 410.

The rotational force transmission unit 510 includes a first pulley member 511 rotatably positioned inside the control body 200 and configured to be rotated by receiving the rotational force of the rotation motor unit 310, a second pulley member 512 rotatably positioned inside the handpiece 100 for skin treatment, and a belt structure 513 having a first end portion wound around the first pulley member 511 and having a second end portion wound around the second pulley member 512.

For example, the first pulley member 511 is mounted on a motor shaft and is configured to be rotated integrally with the motor shaft. Alternatively, the first pulley member 511 may have a structure that is rotated by receiving the rotational force of the rotation motor unit 310 through a reducer including a plurality of gears.

The belt structure 513 has the first end portion wound around the first pulley member 511 inside the control body 200, passes through the inside of the connection cable body 210, and has the second end portion wound around the second pulley member 512 inside the handpiece 100 for skin treatment, thereby transmitting the rotational force of the first pulley member 511 to the second pulley member 512 through endless circulating movement.

For example, the belt structure 513 is manufactured from a flexible material, and may be any one of a wire-type belt, a strip-type belt, a V-belt, and a chain that can be freely bent.

The belt structure 513 is formed of a flexible material and may be any one of a wire-type belt, a strip-type belt, a V-belt, and a chain that can be freely bent. The belt structure 513 passes through the inside of the connection cable body 210 that is bendable so as to secure convenience during treatment, and transmits rotational force of the rotation motor unit 310 to the second pulley member 512 through the first pulley member 511.

The handpiece operating body 400 is the skin stimulation generating unit 410 which is provided in the handpiece 100 for skin treatment and which is configured to generate stimulation on skin. In an embodiment, for example, the handpiece operating body 400 is the ultrasound generating unit 411 configured to generate ultrasound and to irradiate skin with the ultrasound.

In addition to the skin stimulation generating unit 410, the handpiece operating body 400 may include a cam member for generating vibration in the handpiece body casing part 120, a rotating body such as a fan for blowing air, a linearly movable camera or sensor, and so on.

The driving force transmission unit 500 further includes a cartridge driving unit 520 which is positioned in the cartridge housing part 110 and which is configured to linearly move or rotate the handpiece operating body 400 by receiving rotational force from the rotational force transmission unit 510.

An operating shaft of the cartridge driving unit 520 is detachably coupled to an operating shaft of the rotational force transmission unit 510.

When the cartridge housing part 110 is separated from the handpiece body casing part 120, the operating shaft of the cartridge driving unit 520 is separated from the operating shaft of the rotational force transmission unit 510. Furthermore, when the cartridge housing part 110 is coupled to the handpiece body casing part 120, the operating shaft of the cartridge driving unit 520 is coupled to the operating shaft of the rotational force transmission unit 510 and may receive a driving force from the rotational force transmission unit 510.

The cartridge driving unit 520 includes a linear movement conversion unit 521 configured to linearly move the handpiece operating body 400 positioned in the cartridge housing part 110.

The linear movement conversion unit 521 includes a screw member 521a which is rotatably positioned in the handpiece body casing part 120 and which is configured to be rotated by receiving the rotational force of the second pulley member 512, and includes a slider 521b through which the screw member 521a passes and to which the screw member 521a is screw-coupled so that the slider 521b is linearly moved by rotation of the screw member 521a.

The slider 521b is connected to the handpiece operating body 400, i.e., the ultrasound generating unit 411, and linearly reciprocates the ultrasound generating unit 411.

In addition, the linear movement conversion unit 521 further includes a movement guide pin member 521c which is positioned to pass through the slider 521b and which is configured to guide the slider 521b to be moved in a longitudinal direction, and thus linear movement of the handpiece operating body 400 is capable of being stably and smoothly performed.

In addition, the rotational force transmission unit 510 may further include a first rotation direction changing gear member 514 which is mounted on a rotation shaft of the second pulley member 512 and which is configured to transmit the rotational force of the second pulley member 512 to the linear movement conversion unit 521 while the first rotation direction changing gear member 514 changes a rotation direction, and may further include a second rotation direction changing gear member 515 which is mounted on a rotation shaft of the screw member 521a and which is configured to be meshed with and rotated together with the first rotation direction changing gear member 514.

For example, the first rotation direction changing gear member 514 is a first bevel gear, and the second rotation direction changing gear member 515 is a second bevel gear. Although not illustrated in the drawings, the first rotation direction changing gear member 514 and the second rotation direction changing gear member 515 may be provided as a worm gear and a worm wheel gear, respectively, and may also be variously modified and implemented as a known gear structure which changes a rotation direction and which transmits rotational force.

A shaft adapter part 530 for detachably connecting shafts is provided between the rotation shaft of the screw member 521a and the second rotation direction changing gear member 515. The shaft adapter part 530 includes a shaft coupling protrusion part 531 provided on an end portion of any one of the rotation shaft of the screw member 521a and the second rotation direction changing gear member 515, and includes a shaft coupling insertion part 532 which is provided on an end portion of the other one of the rotation shaft of the screw member 521a and the second rotation direction changing gear member 515 and into which the shaft coupling protrusion part 531 is inserted.

For example, the shaft coupling protrusion part 531 is a protrusion having a polygonal cross-sectional shape, and the shaft coupling insertion part 532 is a polygonal insertion groove into which the shaft coupling protrusion part 531 is inserted.

The shaft coupling protrusion part 531 and the shaft coupling insertion part 532 may be variously modified and implemented in other known shapes in which a protrusion is inserted into an insertion part and is caught in a rotation direction to transmit rotational force.

That is, in the linear movement conversion unit 521, when the cartridge housing part 110 is coupled to the handpiece body casing part 120, the rotation shaft of the screw member 521a and the rotation shaft of the second rotation direction changing gear member 515 are connected to each other, and the screw member 521a is rotated by receiving the rotational force of the rotation motor unit 310 in the control body 200. Accordingly, the slider 521b is linearly moved and linearly moves the handpiece operating body 400.

Meanwhile, the skin care device including the lightweight handpiece according to the present disclosure is described below in more detail by using, as an example, a skin ultrasound generating device in which the skin stimulation generating unit 410 that is an example of the handpiece operating body 400 includes the ultrasound generating unit 411 and generates High Intensity Focused Ultrasound (HIFU) by the ultrasound generating unit 411. In addition, the skin care device including the lightweight handpiece according to the present disclosure may be any one of other skin care devices, i.e., a skin laser generating device, a skin RF energy generating device, a negative pressure generating device, and a skin beauty device.

A window part 110a is provided on a lower surface of the cartridge housing part 110 so that ultrasound generated by the ultrasound generating unit 411 which is movably positioned inside the cartridge housing part 110 passes through the window part 110a.

The window part 110a is manufactured from a transparent or translucent film material, and has a known structure manufactured from a film material through which ultrasound can pass, so that a detailed description of the window part 110a is omitted.

The ultrasound generating unit 411 is movably positioned in the cartridge housing part 110, and is, for example, an ultrasound transducer configured to generate High Intensity Focused Ultrasound (HIFU).

For example, the ultrasound generating unit 411 is configured to irradiate a skin muscle layer with High Intensity Focused Ultrasound (HIFU) to generate heat, thereby inducing degeneration and regeneration of skin tissue, reducing skin wrinkles, and increasing skin elasticity.

The ultrasound generating unit 411 is connected to the slider 521b, and linearly reciprocates together with the slider 521b in the cartridge housing part 110 by linear movement of the slider 521b.

The window part 110a is formed such that the window part 110a has a length longer than a movement range of the ultrasound generating unit 411, so that ultrasound generated by the ultrasound generating unit 411 that linearly reciprocates may stably pass through the window part 110a.

The cartridge housing part 110 is filled with a liquid medium for transmitting ultrasound.

The linear movement conversion unit 521 may further include a first bellows member 521d having a first end portion fixed to an inner side surface of the cartridge housing part 110 and having a second end portion fixed to a first end portion of the slider 521b such that the first bellows member 521d is folded and unfolded by movement of the slider 521b, the first bellows member 521d having the screw member 521a positioned therein. Furthermore, the linear movement conversion unit 521 may further include a second bellows member 521e having a second end portion fixed to the inner side surface of the cartridge housing part 110 and having a first end portion fixed to a second end portion of the slider 521b such that the second bellows member 521e is folded and unfolded by movement of the slider 521b, the second bellows member 521e having the screw member 521a positioned therein.

The first bellows member 521d and the second bellows member 521e form sealed spaces blocked from the liquid medium in the cartridge housing part 110, thereby blocking the screw member 521a positioned in the first bellows member 521d and the second bellows member 521e from the medium.

The first bellows member 521d and the second bellows member 521e prevent the liquid medium from leaking to the outside at a shaft coupling portion of the screw member 521a, i.e., at the shaft adapter part 530, when the cartridge housing part 110 is separated from the handpiece body casing part 120.

That is, rotational force generated by the rotation motor unit 310 in the control body 200 is transmitted to the second pulley member 512 by the belt structure 513 passing through the connection cable body 210, and the rotational force transmitted to the second pulley member 512 is transmitted to the screw member 521a through the first bevel gear and the second bevel gear to rotate the screw member 521a.

When the screw member 521a is rotated by receiving the rotational force generated by the rotation motor unit 310 in the control body 200, the slider 521b linearly reciprocates according to a rotation direction of the screw member 521a, and the ultrasound generating unit 411 irradiates the skin with ultrasound while the ultrasound generating unit 411 linearly moves together with the slider 521b.

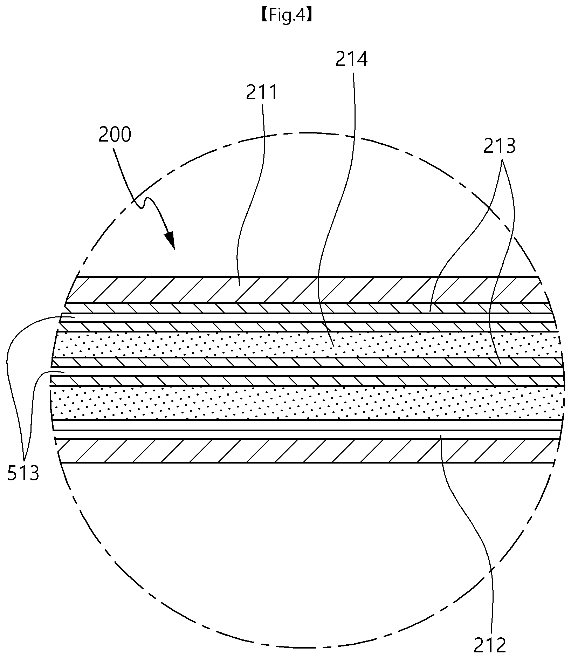

Meanwhile, FIG. 4 is an enlarged view illustrating an embodiment of the connection cable body 210 in an embodiment of the skin care device including the lightweight handpiece according to the present disclosure. Referring to FIG. 4, the rotational force transmission unit 510 further includes a belt movement guide tube part 213 which is fixed inside the connection cable body 210 and through which the belt structure 513 passes so as to guide movement of the belt structure 513.

An operation control cable 212 and the belt structure 513 pass through the inside of the connection cable body 210. The operation control cable 212 is electrically connected to the skin stimulation generating unit 410 positioned in the handpiece body casing part 120, supplies electrical power to the skin stimulation generating unit 410, and controls operation of the skin stimulation generating unit 410. The belt structure 513 transmits rotational force of the driving force generating unit 300.

The belt movement guide tube part 213 is provided as a pair of belt movement guide tube parts 213 through which two lines of the belt structure 513 passing through the connection cable body 210 pass, is fixedly positioned inside the connection cable body 210, and supports positions of the belt structure 513 passing through the belt movement guide tube parts 213. Accordingly, when the connection cable body 210 is bent, a change in tension of the belt structure 513 may be minimized, movement of the belt structure 513 may be smoothly performed, and power transmission through the belt structure 513 may be stably performed.

The belt structure 513 passes through the inside of the pair of belt movement guide tube parts 213 having fixed positions in the connection cable body 210, so that the belt structure 513 may stably transmit the rotational force of the first pulley member 511 to the second pulley member 512.

The connection cable body 210 may include an outer skin part 211 that surrounds the operation control cable 212 and the belt structure 513 that pass through the inside of the connection cable body 210, and may further include a filler 214 formed of an elastic material, the filler 214 being filled in the outer skin part 211 and supporting positions of the operation control cable 212 and the belt movement guide tube parts 213.

For example, the filler 214 is formed of a synthetic resin foam material such as polyethylene foam or the like. The filler 214 stably supports the positions of the belt movement guide tube parts 213 that guide movement of the belt structure 513. Therefore, when the connection cable body 210 is bent, movement of the belt structure 513 may be more smoothly performed, and power transmission through the belt structure 513 may be more stably performed.

According to the present disclosure, the driving force generating unit 300 for operating the operating body in the handpiece is mounted in the control body 200, thereby reducing weight of the handpiece 100 for skin treatment and significantly increasing convenience for a practitioner and satisfaction with the treatment.

In addition, according to the present disclosure, since the structure of the handpiece 100 for skin treatment is simplified, the handpiece 100 for skin treatment may be designed without limitation in size and shape, and freedom in handpiece design may be significantly increased.

While the present disclosure has been described in connection with the embodiments, it is not to be limited thereto but will be defined by the appended claims. In addition, it is to be understood that those skilled in the art can substitute, change or modify the embodiments in various forms without departing from the scope of the present disclosure.

## Claims

1. A skin care device including a lightweight handpiece, the skin care device comprising:
a handpiece for skin treatment, the handpiece being configured to come into contact with skin and to stimulate the skin;
a control body connected to the handpiece for skin treatment by a connection cable body and configured to control operation of the handpiece for skin treatment;
a driving force generating unit mounted inside the control body and configured to generate a driving force;
a handpiece operating body provided in the handpiece for skin treatment and configured to be operated by receiving the driving force of the driving force generating unit; and
a driving force transmission unit which passes through an inside of the connection cable body and which is configured to transmit the driving force of the driving force generating unit to the handpiece operating body.

2. The skin care device of claim 1, wherein the driving force generating unit comprises a rotation motor unit configured to generate rotational force, and
wherein the driving force transmission unit comprises a rotational force transmission unit configured to transmit the rotational force of the rotation motor unit to the handpiece operating body.

3. The skin care device of claim 2, wherein the rotational force transmission unit comprises:
a first pulley member rotatably positioned inside the control body and configured to be rotated by receiving the rotational force of the rotation motor unit;
a second pulley member rotatably positioned inside the handpiece for skin treatment; and
a belt structure having a first end portion wound around the first pulley member and having a second end portion wound around the second pulley member.

4. The skin care device of claim 3, wherein the belt structure is any one of a wire-type belt, a strip-type belt, a V-belt, and a chain.

5. The skin care device of claim 3, wherein the rotational force transmission unit further comprises a belt movement guide tube part which is fixed inside the connection cable body and through which the belt structure passes and moves, the belt movement guide tube part being configured to support a position of the belt structure.

6. The skin care device of claim 5, wherein the connection cable body comprises:
an outer skin part surrounding an operation control cable and the belt structure that pass through the inside of the connection cable body; and
a filler formed of an elastic material, the filler being filled in the outer skin part and supporting positions of the operation control cable and the belt movement guide tube part.

7. The skin care device of claim 2, wherein the handpiece for skin treatment comprises:
a cartridge housing part provided with a skin stimulation generating unit configured to stimulate the skin; and
a handpiece body casing part to which the cartridge housing part is detachably coupled, and
wherein the driving force transmission unit further comprises a cartridge driving unit positioned in the cartridge housing part, the cartridge driving unit being configured to receive the rotational force from the rotational force transmission unit and to linearly move or rotate the handpiece operating body.

8. The skin care device of claim 7, wherein an operating shaft of the cartridge driving unit is detachably coupled to an operating shaft of the rotational force transmission unit.

9. The skin care device of claim 7, wherein the handpiece operating body comprises the skin stimulation generating unit positioned in the cartridge housing part, and
wherein the cartridge driving unit comprises a linear movement conversion unit configured to linearly move the skin stimulation generating unit.

10. The skin care device of claim 9, wherein the linear movement conversion unit comprises:
a screw member rotatably positioned in the handpiece body casing part and configured to be rotated by receiving rotational force of a second pulley member; and
a slider through which the screw member passes and to which the screw member is screw-coupled, the slider being configured to be linearly moved by rotation of the screw member and being connected to the skin stimulation generating unit.

11. The skin care device of claim 10, wherein the linear movement conversion unit further comprises a movement guide pin member which is positioned to pass through the slider and which is configured to guide linear movement of the slider.

12. The skin care device of claim 10, wherein the rotational force transmission unit further comprises a first rotation direction changing gear member and a second rotation direction changing gear member that are configured to change a rotation direction and to transmit the rotational force to the screw member.

13. The skin care device of claim 10, wherein the linear movement conversion unit further comprises:
a first bellows member having a first end portion fixed to an inner side surface of the cartridge housing part and having a second end portion fixed to a first end portion of the slider, the first bellows member being configured to be folded and unfolded by movement of the slider and having the screw member positioned therein; and
a second bellows member having a second end portion fixed to the inner side surface of the cartridge housing part and having a first end portion fixed to a second end portion of the slider, the second bellows member being configured to be folded and unfolded by movement of the slider and having the screw member positioned therein.
